# FASCICULE DE BREVET EUROPEEN

(11) **EP 1 720 588 B1**
(45) Date de publication et mention de la délivrance du brevet: **04.06.2008**
(21) Numéro de dépôt: 05717533.3
(22) Date de dépôt: 02.02.2005
(51) Int. Cl.: A61M 5/158, A61M 25/06

(54) **DISPOSITIF A BEC BASCULANT POUR LA MISE EN PLACE D'UNE CANULE DANS UNE VEINE**
SCHRÄGE HAKENVORRICHTUNG ZUR PLATZIERUNG EINER KANÜLE IN EINER VENE
DROOPED HOOK DEVICE FOR PLACING A CANNULA IN A VEIN

(30) Priorité: 02.03.2004 FR 0402124
(43) Date de publication de la demande: 15.11.2006
(73) Titulaire: VYGON, F-95440 Ecouen (FR)
(72) Inventeur: CARREZ, Jean-Luc, F-95440 Ecouen (FR); DALLE, Valery, F-60270 Gouvieux (FR); GUYOMARC'H, Pierrick, F-95120 Ermont (FR); HUET, Jean-Max, F-92110 Clichy (FR)
(74) Mandataire: Texier, Christian
(86) Numéro de dépôt international: PCT/FR2005/000221
(87) Numéro de publication internationale: WO 2005/094922

(56) Documents cités:
- EP-A- 1 350 538
- WO-A-94/00172
- US-A- 5 147 327
- US-A- 5 183 468
- US-A- 6 077 244
- US-B1- 6 234 999
- US-B2- 6 629 959

## Description

La présente invention concerne les dispositifs utilisés pour la mise en place dans une veine d'une canule constituée d'un cathéter tubulaire court à embase proximale, au moyen d'une aiguille de ponction (voir p. ex. US-A-6 077 244).

La procédure d'introduction comprend une phase de ponction dans laquelle l'aiguille est poussée dans l'embase du cathéter et dans le cathéter en sorte que sa pointe sorte à l'extrémité distale du cathéter et dans laquelle l'opérateur ponctionne avec cette pointe la veine dans laquelle il veut introduire le cathéter, une phase d'introduction dans laquelle l'opérateur fait glisser le cathéter sur l'aiguille en direction distale pour faire pénétrer le cathéter dans la veine, et une phase de retrait dans laquelle l'opérateur retire l'aiguille de la veine, du cathéter et de l'embase du cathéter.

A l'issue de la phase de retrait, la pointe de l'aiguille se trouve à l'air libre et le risque se présente que l'opérateur qui tient le cathéter et son embase d'une main et qui tient l'aiguille de l'autre main, contrôle mal l'aiguille et se pique avec sa pointe.

Pour éviter ce risque, il est connu de fixer provisoirement dans le prolongement vers l'arrière de l'embase du cathéter une cage détachable au travers de laquelle l'aiguille peut coulisser et qui est munie d'un piège pour retenir dans la chambre l'extrémité de ponction de l'aiguille lorsque celle-ci sort de l'embase et pour rester en place sur cette extrémité lorsque la cage est détachée de l'embase.

Pour fixer provisoirement la cage sur l'embase de l'aiguille, il est connu de réaliser un emboîtement conique à friction de la cage dans ou sur l'embase de l'aiguille, en sorte que la cage se détache de l'embase sous l'effet d'une traction exercée axialement sur l'aiguille après que l'extrémité de ponction de l'aiguille soit arrivée dans la cage (EP 0 456 694 ou US 5 322 517, US 5 135 504, US 5 176 655, et autres).

Le risque subsiste toutefois que la cage se détache de l'embase prématurément avant que l'extrémité de ponction de l'aiguille soit piégée dans la cage.

Pour éviter ce risque, il a été préconisé de munir la cage d'un crochet mobile transversalement maintenu par l'aiguille dans une position de retenue où il est en prise avec l'embase du cathéter, et apte à venir de lui-même dans une position de libération lorsque l'extrémité de ponction de l'aiguille est retirée dans la cage.

La publication EP 0 891 198 ou US 6 001 080 réalise cette retenue par pénétration, dans une cavité formée sur la face interne de la paroi de l'embase du cathéter, d'un bec de la cage, ledit bec étant maintenu en position de retenue par un contact latéral avec l'aiguille et se trouvant libéré et apte à se déplacer transversalement dans la cage pour échapper à la cavité lorsque ce contact latéral est supprimé par le retrait de l'aiguille en arrière du bec.

Ce dispositif de retenue, entièrement caché dans l'embase et dans la cage, est difficile à contrôler et le risque existe que le déplacement radial automatique du bec soit insuffisant pour libérer la cage de l'embase.

La publication US 6 234 999 décrit un autre dispositif de retenue dans lequel la cage présente un organe de crochetage externe retenu par une collerette externe de l'embase mais qui n'est pas maintenu par l'aiguille, en sorte qu'une traction intempestive sur la cage risque de supprimer prématurément la retenue.

La publication US 6 629 959 B2 décrit des systèmes à ressort, contraints qui se déclenchent au retrait de l'aiguille et un système de verrouillage cage/canule.

La publication EP 1 350 538 A1 décrit un système à ressort contraint pour une aiguille seule.

La publication US 5 147 327 décrit un système pour aiguille seule à fourreau glissant sur un tube métallique.

La publication 5 183 468 décrit une cage avec un levier qui bascule au sortir de l'aiguille autorisant un serrage autour de l'aiguille.

La publication PCT WO 94/00172 décrit un système à fourreau flexible et à élément ressort précontraint.

La présente invention a pour but de fournir une cage muni d'un dispositif de retenue affranchi des inconvénients précités et notamment un dispositif de retenu qui soit à la fois apparent à l'extérieur de la cage et de l'embase et maintenu par l'aiguille en position de retenue tant que l'extrémité de ponction de l'aiguille n'est pas retirée dans la cage.

Un objet de l'invention est donc un dispositif pour la mise en place dans une veine d'une canule constituée d'un cathéter court à embase proximale, ce dispositif comprenant une aiguille qui présente une extrémité de ponction et une cage anti-pique qui prolonge l'embase en direction proximale, cette cage déterminant une chambre traversée à coulisse par l'aiguille et munie d'un piège pour retenir dans la chambre l'extrémité de ponction de l'aiguille lorsque l'aiguille est retirée de l'embase du cathéter, la cage et l'embase étant munis de moyens de retenue coopérant pour assurer une retenue provisoire de la cage et de l'embase avant que l'extrémité de ponction de l'aiguille soit piégée dans la chambre de la cage, lesdits moyens de retenue comprenant un rebord externe formé sur l'embase et un bec externe prévu sur la cage pour être retenu par ce rebord, caractérisé en ce que ce bec est formé à une extrémité d'un organe de retenue monté à bascule sur la cage autour d'un axe de pivotement transversal à la direction de coulissement de l'aiguille, ledit organe présentant une extrémité opposée en contact latéral avec l'aiguille lorsque celle-ci traverse la cage et ledit organe étant conçu pour basculer autour dudit axe lorsqu'il n'est plus au contact de l'aiguille, en sorte que le bec se soulève et se dégage du rebord de l'embase tandis que ladite extrémité opposée de l'organe s'abaisse et s'interpose devant l'extrémité de ponction de l'aiguille, empêchant que cette extrémité puisse ressortir de la chambre par la sortie distale de la chambre.

Dans des réalisations particulières, le dispositif de l'invention présente encore une ou plusieurs des caractéristiques suivantes :
- l'organe de retenue est conçu en sorte que le poids de la partie de l'organe de retenue située entre l'axe de pivotement et l'extrémité de retenue soit inférieur au poids de la partie de l'organe située entre cet axe et ladite extrémité ;
- l'axe de pivotement est constitué par des tétons latéraux formés sur l'organe de retenue et qui sont reçus à pivotement dans un berceau constitué par découpage de deux parois opposées formées sur la cage de part et d'autre du l'organe de retenue ;
- le berceau reçoit les tétons par encliquetage ;
- l'organe de retenue présente une languette souple qui est maintenue comprimée élastiquement par une paroi de la cage lorsque l'organe est maintenue par l'aiguille et qui se déploie sous cette paroi lorsque l'organe a basculé sous l'effet du retrait de l'aiguille, en sorte qu'un basculement de l'organe en sens inverse est empêché par butée de cette languette sous ladite paroi.

On décrira ci-après à titre d'exemple une réalisation d'un dispositif conforme à l'invention en référence aux figures des dessins joints sur lesquels :
- la figure 1 est une vue en perspective extérieure du dispositif dans laquelle le cathéter court avec son embase, l'aiguille, la cage et l'organe de retenue ont été représentés séparés ;
- la figure 2 est une vue partielle en perspective avec coupe du dispositif où l'on voit également l'embase de l'aiguille, l'organe de retenue étant en position de retenue de la cage sur l'embase ;
- la figure 3 est une coupe longitudinale du dispositif de la figure 2 ;
- les figures 4 et 5 sont des vues en perspective avec coupe partielle du dispositif lors du retrait de l'aguille ;
- les figures 6 et 7 sont des vues en perspective et en coupe du dispositif lorsque l'organe de retenue a basculé, et
- les figures 8 et 9 sont des vues en perspective et en coupe du dispositif lorsque la cage est détachée de l'embase du cathéter. Le dispositif représenté sur les figures comprend :
   - un cathéter court (1) muni d'une embase proximale (2) ;
   - une aiguille de ponction (3) munie d'une embase proximale (4) ;
   - une cage de protection (5) munie d'un organe de retenue (6).

De façon en soi connue, la cage détermine une chambre (7) de passage d'aiguille qui présente une entrée proximale (7a) et une sortie distale (7b) et autour de la sortie distale (7b), la cage forme un nez (8) apte à s'emboîter avec ou sans friction dans l'entrée (2a) de l'embase (2) du cathéter court.

L'organe de retenue (6), par exemple venu de moulage en résine de synthèse, présente :
- une extrémité de retenue (6a),
- une extrémité opposée (6b) de contact latéral avec l'aiguille,
- deux tétons transversaux coaxiaux (6c),
- une languette souple (6d).

Les deux tétons forment un axe de pivotement et sont aptes à être reçus par encliquetage dans un berceau (9) formé par découpage de deux parois opposées (10, 11) que présente la cage (5) et entre lesquelles peut basculer l'organe de retenue (6).

L'embase du cathéter présente vers son entrée une collerette externe (12) continue ou discontinue avec laquelle l'extrémité de retenue du levier peut venir en prise.

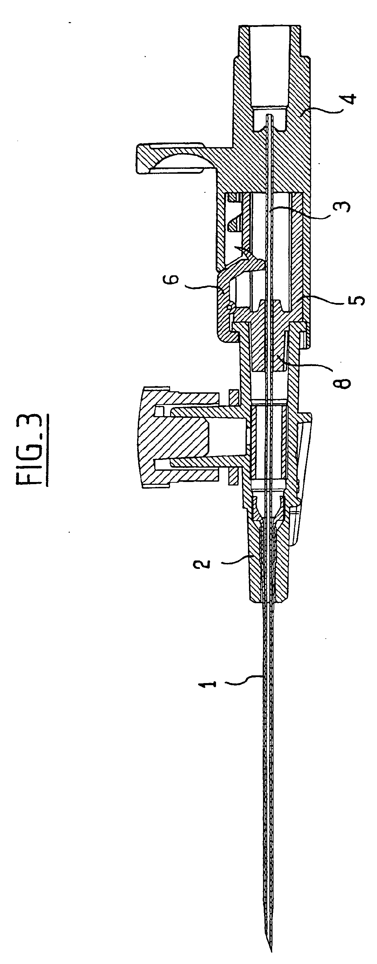

Initialement (figure 3), l'aiguille traverse la cage, traverse la canule et ressort à l'extrémité du tube cathéter ; dans cette situation, la cage est maintenue entre l'embase du cathéter où est reçu le nez de la cage et l'embase (4) de l'aiguille, tandis que l'organe de retenue (6) est maintenu en position de retenue par l'aiguille.

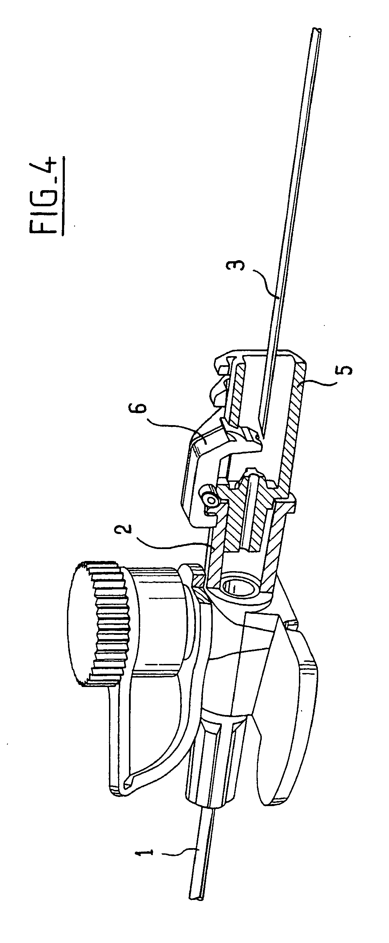

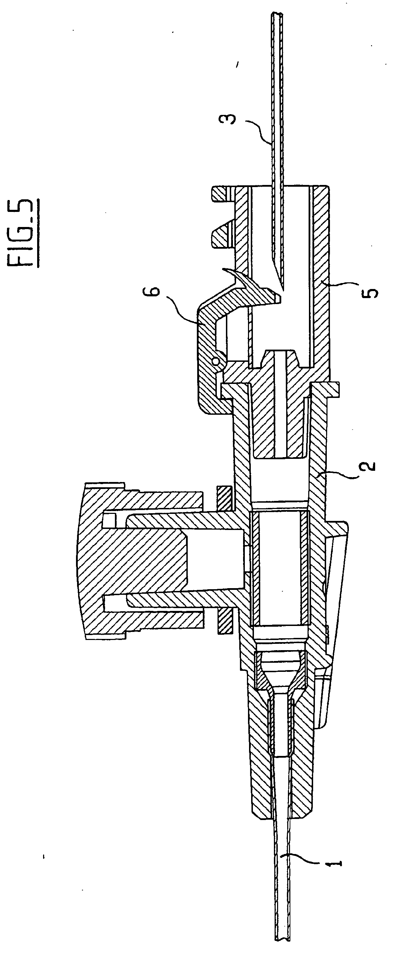

Après la ponction et la mise en place du cathéter dans la veine, l'aiguille est retirée et son extrémité arrive dans la cage en retrait de l'organe (figures 4 et 5).

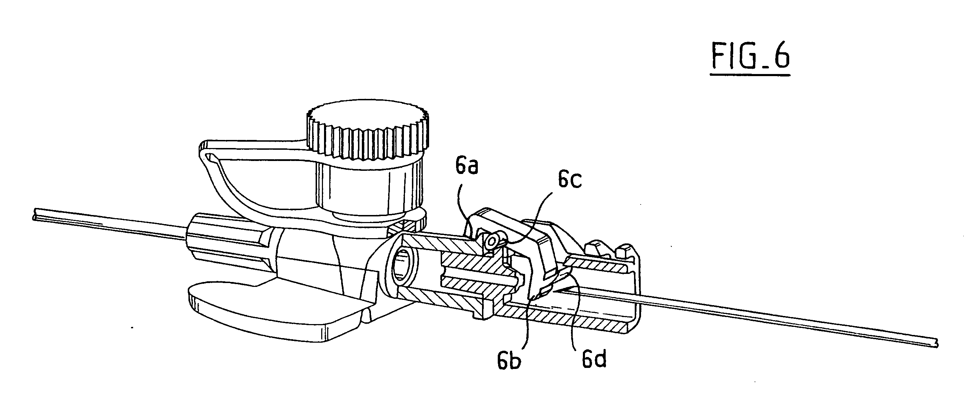

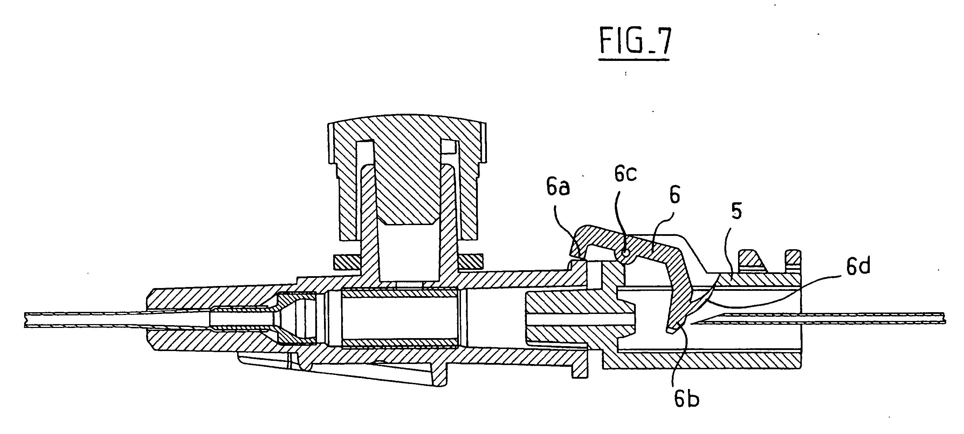

L'organe qui n'est plus maintenu par l'aiguille bascule de lui-même (figures 6 et 7), et sa languette souple (6d) vient se déployer sous la paroi latérale de la cage (5), empêchant un basculement inverse de l'organe.

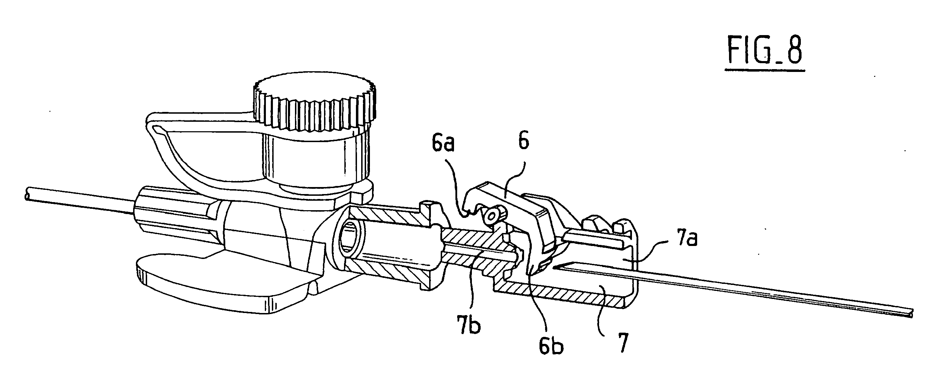

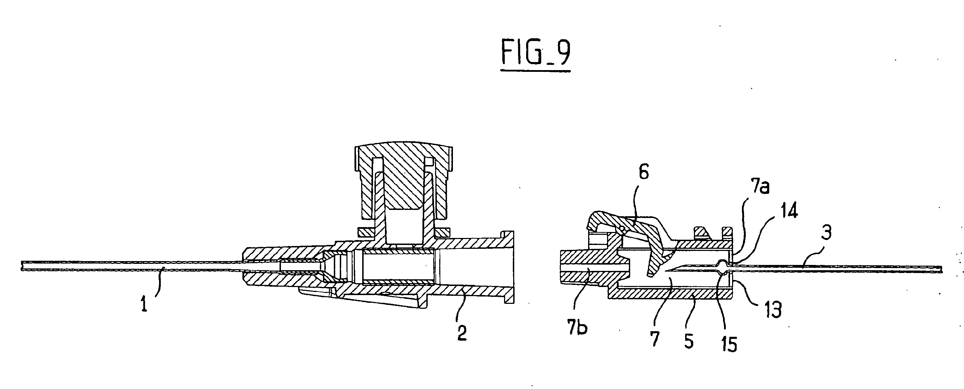

L'extrémité (6b) de l'organe se trouve interposée entre l'aiguille et la sortie (7b) de la chambre (7) empêchant que l'aiguille puisse ressortir de la cage par cette sortie (figures 8 et 9).

De préférence, des moyens sont également prévus de façon en soi connue, pour empêcher que l'extrémité de ponction de l'aiguille puisse sortir de la cage par l'entrée proximale de la chambre.

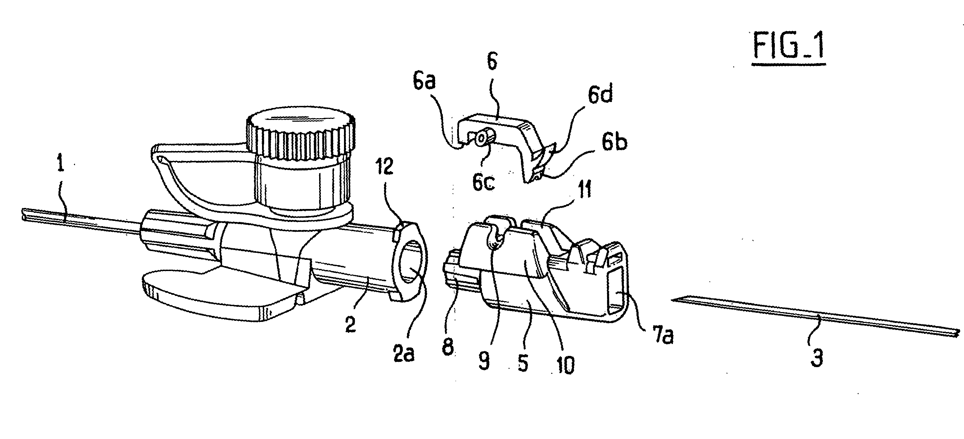

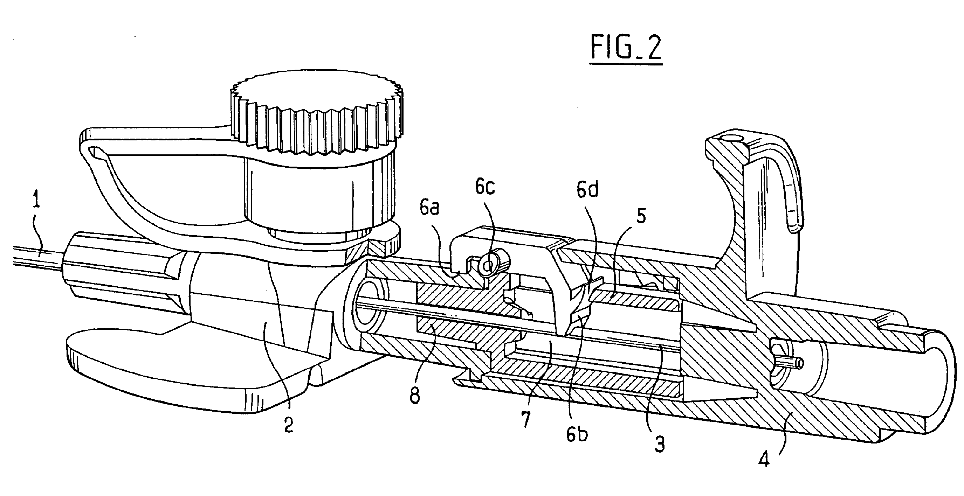

Ces moyens, dont on connaît de nombreux exemples n'ont pas été représentés sur les figures 1 à 8 pour ne pas charger les figures.

On a proposé de relier la cage à l'embase de l'aiguille par une liaison déployable telle qu'à l'état déployée, la longueur de la liaison soit inférieure à la longueur de l'aiguille (WO 94/00172, US 5 176 655, US 6 234 999, US 6 001 080).

On a proposé également de munir l'entrée proximale de la cage d'une paroi transversale pourvue d'un trou pour le passage de l'aiguille et de munir l'aiguille d'un renflement local en avant de ce trou en direction de l'embase du cathéter en sorte que le coulissement de l'aiguille en direction proximale soit arrêté par butée de ce renflement contre le pourtour du trou.

La présente invention ne porte pas sur un choix particulier d'un tel dispositif de retenue et, pour l'exemple uniquement, on a représenté sur la figure 9 un dispositif constitué par une paroi transversale fixe (13) munie d'un trou (14) pour arrêter un épanouissement local (15) de l'aiguille.

L'invention n'est pas limitée aux réalisations qui ont été décrites.

## Revendications

1. Dispositif pour la mise en place dans une veine d'une canule constituée d'un cathéter tubulaire court (1) à embase proximale (2), ce dispositif comprenant une aiguille (3) qui présente une extrémité (3a) de ponction et une cage anti-pique (5) qui prolonge l'embase en direction proximale, cette cage déterminant une chambre (7) traversée à coulisse par l'aiguille d'une entrée proximale (7a) à une sortie distale (7b) de la chambre et munie d'un piège pour retenir dans la chambre l'extrémité de ponction de l'aiguille lorsque l'aiguille est retirée de l'embase du cathéter, la cage et l'embase étant munis de moyens de retenue coopérant pour assurer une retenue provisoire de la cage sur l'embase du cathéter avant que l'extrémité de ponction de l'aiguille soit piégée dans la chambre de la cage, lesdits moyens de retenue comprenant un rebord externe (12) formé sur l'embase et un bec externe (6a) prévu sur la cage pour être retenue par ce rebord (12), **caractérisé en ce que** ce bec est formé à une extrémité (6a) d'un organe de retenue (6) monté à bascule sur la cage autour d'un axe de pivotement transversal à la direction de coulissement de l'aiguille, ledit organe présentant une extrémité opposée (6b) en contact latéral avec l'aiguille lorsque celle-ci traverse la cage et ledit organe étant conçu pour basculer autour dudit axe lorsqu'il n'est plus au contact de l'aiguille, en sorte que le bec se soulève et se dégage du rebord de l'embase tandis que ladite extrémité opposée de l'organe s'abaisse et s'interpose devant l'extrémité de ponction de l'aiguille, empêchant que cette extrémité puisse ressortir de la chambre par la sortie distale (7b) de la chambre.

2. Dispositif selon la revendication 1 dans lequel l'organe de retenue (6) est conçu en sorte que le poids de la partie de l'organe située entre l'axe de pivotement et l'extrémité de retenue (6a) soit inférieur au poids de la partie de l'organe située entre cet axe et ladite extrémité de contact (6b).

3. Dispositif selon la revendication 1 ou 2 dans lequel ledit axe de pivotement est constitué par des tétons latéraux (6c) formés sur l'organe et qui sont reçus dans un berceau (9) constitué par découpage de deux parois opposées (10 ; 11) formées sur la cage de part et d'autre de l'organe de retenue.

4. Dispositif selon la revendication 3 dans lequel ledit berceau (9) reçoit lesdits tétons (6c) par encliquetage.

5. Dispositif selon l'une des revendications 1 à 4 dans lequel ledit organe de retenue (6) présente une languette souple (6d) qui est maintenue comprimée élastiquement par une paroi de la cage lorsque l'organe est maintenue par l'aiguille et qui se déploie sous cette paroi lorsque le levier a basculé sous l'effet du retrait de l'aiguille, en sorte qu'un basculement de l'organe en sens inverse est empêché par butée de cette languette sous ladite paroi.

6. Dispositif selon l'une des revendications 1 à 5 et qui comporte des moyens (13 ; 15) pour empêcher que l'extrémité de ponction de l'aiguille puisse sortir de la cage par l'entrée proximale (7a) de la chambre.

## Claims

1. An arrangement for the insertion into a vein of a cannula composed of a short tubular catheter (1) with a proximal base (2), this arrangement including a needle (3) which has a skin-puncture end (3a) and anti-prick cage (5) which extends the base in the proximal direction, this chamber forming a chamber (7) through which the needle slides from a proximal entrance (7a) to a distal exit (7b) of the chamber, and is equipped with a trap to hold the puncture end of the needle in the chamber when the needle is withdrawn from the catheter base, the cage and the base being equipped with retention means that combine so as to perform temporary retention of the cage on the catheter base before the puncture end of the needle is trapped in the chamber of the cage, the said retention means including an external rim (12) formed on the base and an external nose (6a) provided on the cage to be held by this rim (12), **characterised in that** this nose is formed at one end (6a) of a retention device (6) mounted to tilt in the cage around a pivoting axis which is transverse to the sliding direction of the needle, the said device having one opposite end (6b) in lateral contact with the needle when the latter traverses the cage and the said device being designed to tilt around the said axis when it is no longer in contact with the needle, so that the nose lifts and releases itself from the rim of the base while the said opposite end of the device drops and positions itself in front of the puncture end of the needle, preventing this end from exiting from the chamber via the distal end (7b) of the chamber.

2. The arrangement according to claim 1, wherein the retention device (6) is designed so that the weight of the part of the device located between the pivoting axis and the retention end (6a) is less than the weight of the part of the device located between this axis and the said contact end (6b).

3. The arrangement according to claim 1 or 2, wherein the said pivoting axis is composed of lateral nipples (6c) formed on the device, and which are accommodated in a cradle (9) created by cut-outs in two opposite walls (10, 11) formed on the cage on either side of the retention device.

4. The arrangement according to claim 3, wherein the said cradle (9) accommodates the said nipples (6c) by a click-on action.

5. The arrangement according to any one of claims 1 to 4, wherein the said retention device (6) has a flexible tongue (6d) which is held compressed elastically by a wall of the cage when the device is held by the needle, and which deploys under this wall when the lever has tilted as a result of withdrawing the needle, so that tilting of the device in the reverse direction is prevented by trapping this tongue under the said wall.

6. The arrangement according to any one of claims 1 to 5, and which includes means (13; 15) to prevent the puncture end of the needle from leaving the cage via the proximal entrance (7a) of the chamber.

## Patentansprüche

1. Vorrichtung zum Platzieren einer Kanüle, die von einem rohrförmigen, kurzen Katheter (1) mit proximalem Ansatz (2) gebildet ist, in einer Vene, wobei diese Vorrichtung eine Nadel (3), die ein Punktierungsende (3a)aufweist, und einen Stechschutzkäfig (5), der den Ansatz in proximaler Richtung verlängert, umfasst, dieser Käfig eine Kammer (7) festlegt, die gleitend von der Nadel von einem proximalen Eintritt (7a) zu einem distalen Austritt (7b) der Kammer durchsetzt wird, und mit einer Fangeinrichtung versehen ist, um in der Kammer das Punktierungsende der Nadel zurückzuhalten, wenn die Nadel aus dem Ansatz des Katheters herausgezogen wird, der Käfig und der Ansatz mit Haltemitteln versehen sind, die zusammenwirken, um einen vorläufigen Halt des Käfigs auf dem Ansatz des Katheters sicherzustellen, bevor das Punktierungsende der Nadel in der Kammer des Käfigs gefangen wird, und die genannten Haltemittel einen äußeren Rand (12), der auf dem Ansatz ausgebildet ist, und eine äußere Nase (6a) umfassen, die auf dem Käfig vorgesehen ist, um von diesem Rand (12) zurückgehalten zu werden, **dadurch gekennzeichnet, dass** diese Nase an einem Ende (6a) eines Halteorgans (6) ausgebildet ist, das schwenkbar um eine zur Richtung der Gleitbewegung der Nadel querverlaufende Schwenkachse auf dem Käfig angebracht ist, wobei das genannte Organ eine entgegengesetztes Ende (6b) aufweist, das in seitlicher Berührung mit der Nadel steht, wenn diese den Käfig durchsetzt, und das genannte Organ so konzipiert ist, dass es um die genannte Achse schwenkt, wenn es nicht mehr in Berührung mit der Nadel steht, derart, dass sich die Nase anhebt und sich vom Rand des Ansatzes löst, während das genannte entgegengesetzte Ende des Organs sich absenkt und sich vor dem Punktierungsende der Nadel dazwischenlegt, wobei es verhindert, dass dieses Ende aus der Kammer durch den distalen Austritt (7b) der Kammer hervortreten kann.

2. Vorrichtung nach Anspruch 1, worin das Halteorgan (6) derart konzipiert ist, dass das Gewicht des Teiles des Organs, das zwischen der Schwenkachse und dem Halteende (6a) gelegen ist, kleiner ist als das Gewicht des Teiles des Organs, das zwischen dieser Achse und dem genannten Berührungsende (6b) gelegen ist.

3. Vorrichtung nach Anspruch 1 oder 2, worin die genannte Schwenkachse aus seitlichen Ansatzkuppen (6c) gebildet ist, die auf dem Organ ausgebildet sind und die in einer Wiege (9) aufgenommen sind, die durch Abschneiden zweier gegenüberliegender Wände (10; 11) gebildet ist, die auf dem Käfig beiderseits des Halteorgans ausgebildet sind.

4. Vorrichtung nach Anspruch 3, worin die genannte Wiege (9) die genannten Ansatzkuppen (6c) durch Einrasten aufnimmt.

5. Vorrichtung nach einem der Ansprüche 1 bis 4, worin das genannte Halteorgan (6) eine weiche Zunge (6d) aufweist, die elastisch zusammengedrückt durch eine Wand des Käfigs gehalten wird, wenn das Organ durch die Nadel gehalten ist, und die sich unter dieser Wand ausspreizt, wenn der Hebel unter Wirkung des Zurückziehens der Nadel verschwenkt wurde, und zwar derart, dass ein Verschwenken des Organs in umgekehrter Richtung durch das Anliegen dieser Zunge unter der genannten Wand verhindert wird.

6. Vorrichtung nach einem der Ansprüche 1 bis 5, und die Mittel (13; 15) umfasst, um zu verhindern, dass das Punktierungsende der Nadel den proximalen Eintritt (7a) der Kammer verlassen kann.
